# EUROPEAN PATENT APPLICATION

(11) **EP 2 083 076 A1**
(43) Date of publication of application: **29.07.2009**
(21) Application number: 07828288.6
(22) Date of filing: 21.09.2007
(51) Int. Cl.: C12N 15/09, A61K 35/14, A61P 35/00, A61P 37/02, A61P 43/00, C12N 5/10, C12N 15/00

(54) **IN VITRO DIFFERENTIATION/INDUCTION OF LYMPHOCYTE FROM STEM CELL HAVING GENOTYPE PROVIDED AFTER GENE RECONSTITUTION**

(30) Priority: 25.09.2006 JP 2006259295
(71) Applicant: Riken, Wakou-shi, Saitama 351-0198 (JP)
(72) Inventor: WAKAO, Hiroshi, Yokohama-shi Kanagawa 230-0045 (JP); FUJII, Shin-ichiro, Yokohama-shi Kanagawa 230-0045 (JP); SHIMIZU, Kanako, Yokohama-shi Kanagawa 230-0045 (JP); KOSEKI, Haruhiko, Yokohama-shi Kanagawa 230-0045 (JP); TANIGUCHI, Masaru, Yokohama-shi Kanagawa 230-0045 (JP); OGURA, Atsuo, Tsukuba-shi Ibaraki 305-0074 (JP); KAWAMOTO, Hiroshi, Yokohama-shi Kanagawa 230-0045 (JP)
(74) Representative: Woods, Geoffrey Corlett
(86) International application number: PCT/JP2007/068335
(87) International publication number: WO 2008/038579

(57) **Abstract**

The present invention provides a production method of a functional differentiated cell having a post-rearrangement genotype of a particular antigen receptor gene, which includes culturing a stem cell having the genotype in a medium to give the differentiated cell derived from the stem cell. As the stem cell having the genotype, a stem cell (e.g., ES cell) established by transplantation of the nucleus of a cell having the genotype is preferable. As the differentiated cell, NKT cell is preferable.

## Description

### Technical Field

The present invention provides a production method of a functional cell, for example, NKT cell.

### Background Art

Natural killer (NK) T cell is an immunocyte producing various cytokines and having an immunoregulatory function. Utilizing such property, therefore, application to the treatment of cancer or autoimmune diseases has been considered. However, due to its trace amount present, it is extremely difficult to provide an experimentally sufficient amount of the cell. Even if NKT cells collected from the body could be grown ex vivo, when the cells have a functional defect, the cells cannot be used for treatment and the like. Thus, there is a demand for the development of a method capable of producing functional NKT cells in vitro in an amount sufficient for basic study or treatments. It is considered that a sufficient amount of object cells will be obtained if NKT cells could be produced from embryonic stem cells (ES cells). Although a method of introducing differentiation of ES cells into T cells has been reported (patent document 1 and non-patent document 1), the reported method cannot produce NKT cells.

The present inventors previously reported a production method of a clone animal by the use of NKT cell as a donor cell, a clone mammal obtained by the method, a method of obtaining embryonic stem (ES) cells from the embryo of the clone animal, and ES cells obtained by the method (patent document 2).
patent document 1: US-A-20040171148
patent document 2: WO2006/018998
non-patent document 1: Schmitt et al., Nature Immunology, vol. 5, p. 410-417 (2004)

### Disclosure of the Invention

### Problems to be Solved by the Invention

The present invention aims to provide a method of producing functional cells such as NKT cells and the like with high efficiency.

### Means of Solving the Problems

The present inventors have conducted intensive studies and found that, using a stem cell having a post-rearrangement genotype of a particular antigen receptor gene, a particular cell having the genotype, for example, NKT cells, can be produced in vitro in a large amount with high efficiency, which resulted in the completion of the present invention. Accordingly, the present invention provides the following.
[1] A method of producing a functional differentiated cell having a post-rearrangement genotype of a particular antigen receptor gene, which comprises culturing a stem cell having the genotype in a medium to give a differentiated cell derived from the stem cell.
[2] The method of the above-mentioned [1], wherein the stem cell having the genotype is established by transplanting the nucleus of a cell having the genotype.
[3] The method of the above-mentioned [1] or [2], wherein the stem cell is an ES cell.
[4] The method of any of the above-mentioned [1] to [3], wherein the antigen receptor is a T cell receptor.
[5] The method of the above-mentioned [4], wherein the T cell receptor is an NKT cell specific T cell receptor.
[6] The method of the above-mentioned [1], wherein an ES cell established by transplanting the nucleus of an NKT cell to an enucleated oocyte is cultured in a medium to give a cell of NKT cell lineage.
[7] The method of the above-mentioned [6], wherein the cell of NKT cell lineage is an NKT cell.
[8] The method of the above-mentioned [1] - [7], wherein the culture is performed in the presence of a feeder cell.
[9] The method of the above-mentioned [8], wherein the feeder cell expresses a Notch ligand.
[10] The method of the above-mentioned [9], wherein the Notch ligand is a delta family member.
[11] The method of the above-mentioned [10], wherein the delta family member is Dlk1.
[12] The method of any of the above-mentioned [8] to [11], wherein the culture is performed using one or more factors selected from the group consisting of Flt3 ligand, IL-7 and IL-15.
[13] A differentiated cell obtainable by the method of any of the above-mentioned [1] to [12].
[14] The cell of the above-mentioned [13], wherein the differentiated cell is an NKT cell.

### Effect of the Invention

According to the method of the present invention, a differentiated cell (e.g., NKT cell) can be produced in vitro in a large amount with high efficiency from a stem cell having a post-rearrangement genotype of a particular antigen receptor gene. The method of the present invention also offers advantages in that immune rejection can be avoided during transplantation of a cell obtainable by the method of the present invention since a stem cell derived from an individual undergoing the transplantation can be used, and the like.

### Brief Description of the Drawings

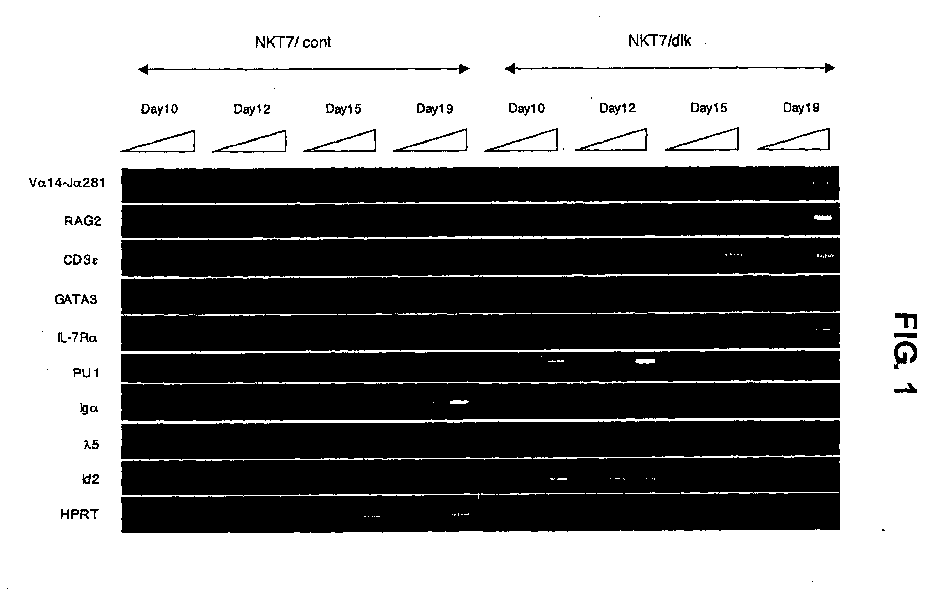

Fig. 1 shows expression of various mRNAs by ntES cells cultured on OP9 cells or OP9-dlk cells. NKT7/cont: ntES cells (clone 7) cultured on OP9 cells, NKT7/dlk: ntES cells (clone 7) cultured on OP9-dlk cells

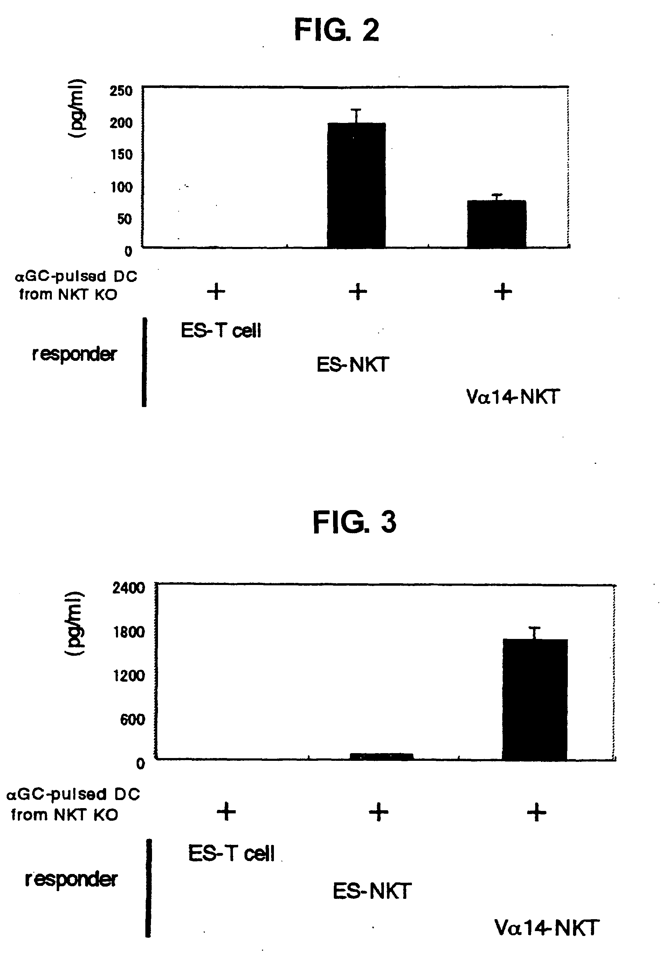

Fig. 2 shows concentrations of IFNγ in a culture medium, which was released by TCRVβ⁺/αGalCer-CD1d dimer⁺ cells cocultured with αGalCer-presenting DC. ES-T cell: DP cells differentiated from conventional ES cells, ES-NKT: NKT cells defined by TCRVβ⁺/αGalCer-CD1d dimer⁺ differentiated from ntES cells, Vα14-NKT: NKT cells purified from mouse
Fig. 3 shows concentrations of IL-4 in a culture medium, which was released by TCRVβ⁺/αGalCer-CD1d dimer⁺ cells cocultured with αGalCer-presenting DC. The abbreviations are the same as in Fig. 2.

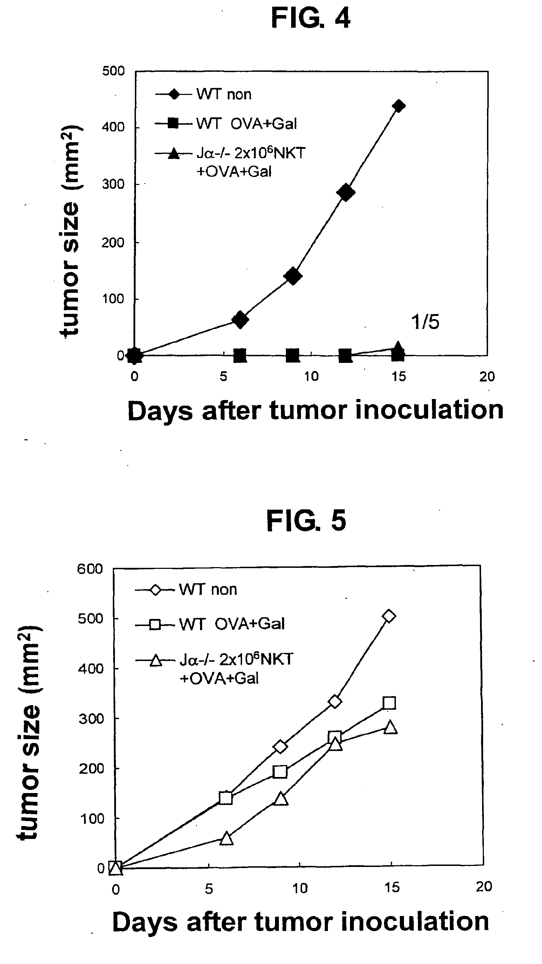

Fig. 4 shows changes in tumor diameter when OVA and α-GalCer were administered to NKT cell deficient mouse after transfusion of TCRVβ⁺/αGalCer-CD1d dimer⁺ cell, and EG7 lymphoma cells were subcutaneously administered to the mouse one week later. WT non: EG7 lymphoma cells were subcutaneously administered to C57BL/6 mouse, WT OVA+Gal: OVA and α-GalCer were administered to C57BL/6 mouse, and EG7 lymphoma cells were subcutaneously administered to the mouse one week later, Jα-/-2×10⁶ NKT+OVA+Gal: TCRVβ⁺/aGalCer-CD1d dimer⁺ cells were transfused to NKT cell deficient mouse, OVA and α-GalCer were administered, and EG7 lymphoma cells were subcutaneously administered to the mouse one week later
Fig. 5 shows changes in tumor diameter when OVA and α-GalCer were administered to NKT cell deficient mouse after transfusion of TCRVβ⁺/αGalCer-CD1d dimer⁺ cells, and EL4 lymphoma cells were subcutaneously administered to the mouse one week later. WT non: EL4 lymphoma cells were subcutaneously administered to C57BL/6 mouse, WT OVA+Gal: OVA and α-GalCer were administered to C57BL/6 mouse, and EL4 lymphoma cells were subcutaneously administered to the mouse one week later, Jα-/-2×10⁶ NKT+OVA+Gal: TCRVβ⁺/αGalCer-CD1d dimer⁺ cells were transfused to NKT cell deficient mouse, OVA and α-GalCer were administered, and EL4 lymphoma cells were subcutaneously administered to the mouse one week later

### Best Mode for Carrying out the Invention

The present invention provides an in vitro production method of a functional cell, and a cell obtained thereby. The method of the present invention may comprise culturing, in a medium, a stem cell having a post-rearrangement genotype of a particular antigen receptor gene (e.g., B cell receptor, T cell receptor) to give a differentiated cell derived from the stem cell.

The stem cell to be used in the present invention is not particularly limited as long as it has a post-rearrangement genotype of a particular antigen receptor gene and may be, for example, a stem cell established by nuclear transplantation or a somatic cell genetically-modified to become an ES cell (Takahashi and Yamanaka, Cell 126: 663-676 (2006)). Examples of the stem cell include ES cell and somatic stem cells such as hematopoietic cell and the like, with preference given to ES cell. When the stem cell having a post-rearrangement genotype of a particular antigen receptor gene is a stem cell established by nuclear transplantation, such stem cell can be produced by a method known per se. For example, such stem cell can be produced by transplanting the nucleus of a cell having a post-rearrangement genotype of a particular antigen receptor gene to an enucleated cell (e.g., oocyte), and subjecting the cell to a predetermined operation (see, for example, Wakayama et al., Nature 394: 369-374 (1998); Inoue et al., Biol. Reprod. 69: 1394-1400 (2003); WO2006/018998).

When the stem cell is established by nuclear transplantation, the cell from which the nucleus is to be extracted is not particularly limited as long as the rearrangement of antigen receptor gene has been completed. It may be an antigen receptor expressing cell. Examples of such cell include B cells, T cells and NKT cells. The cell from which the nucleus is to be extracted may also be one derived from the peripheral blood.

The stem cell having a post-rearrangement genotype of a particular antigen receptor gene may be a cell derived from any mammal species. Examples of the mammal species include primates such as human, monkey and the like, rodents such as mouse, rat, hamster, guinea pig and the like, rabbit, cat, dog, horse, bovine, sheep, goat and swine. When the stem cell is established by nuclear transplantation, it may also be established by transplanting the nucleus of a cell having a post-rearrangement genotype of a particular antigen receptor gene to an enucleated cell derived from a mammal of the same species with the nucleus, or established by transplanting the nucleus to an enucleated cell derived from a mammal of a different species from the nucleus.

Preferably, the stem cell having a post-rearrangement genotype of a particular antigen receptor gene may be a stem cell having the nucleus of an immunocyte after completion of the rearrangement of antigen receptor gene, and more preferably, an ES cell having the nucleus of NKT cell. In this case, as the stem cell, one disclosed in WO2006/018998 can be preferably used.

NKT cell is one kind of lymphocytes having a regulatory function in the immune system, though it is present in a small ratio. NKT cell has two antigen receptors of T cell receptor (TCR) and NK receptor. NKT cell expresses a specific repertoire different from that of conventional T cells and NK cells. For example, as to the mouse β chain, not less than 90% of NKT cells expresses a limited repertoire of mostly Vβ8, and additionally Vβ7 and Vβ2, and as to the α chain, it expresses uniform Vα14-Jα281. The uniform TCRα chain is constructed as a result of selection of Vα14 gene and Jα281 gene from V and J gene groups and rearrangement thereof on the genome, during rearrangement of the TCR gene (Taniguchi et al., Annu Rev Immunol 21: 483-513 (2003)). In human, it is known to be a combination of non-polymorphic Vα24 having high homology with mouse Vα14, and Vβ11 closely related to Vβ8.2.

The medium can be prepared using a medium used for culturing animal cells as a basal medium. The basal medium is not particularly limited as long as it can be used for culture of animal cells and may be Eagle MEM medium, αMEM medium, DMEM medium, ham medium, RPMI 1640 medium, Fischer's medium, a mixed medium thereof and the like. The medium may also contain a serum or a serum replacement. The medium can also contain fatty acid or lipid, amino acid (e.g., non-essential amino acid), vitamin, growth factor, cytokine, antioxidant, 2-mercaptoethanol, pyruvic acid, buffers, inorganic salts and the like.

A stem cell can be cultured in the presence or absence of a feeder cell. When NKT cell is to be produced, for example, it can be cultured in the presence of a feeder cell (e.g., stroma cell such as OP-9 cell and the like). The feeder cell can be a primary cultured cell or cell line. The mammalian species from which the feeder cell derives may be the same as the mammalian species from which the stem cell derives, and it is also preferable that both the feeder cell and stem cell be derived from the same animal species. The feeder cells may also be modified genetically. For example, a gene may be exogenously introduced into a feeder cell so that a desired factor can be expressed, or the expression of the factor can be enhanced. The gene to be introduced can be appropriately selected according to the kind of the cells to be produced. For example, the gene to be introduced may be a Notch ligand.

Notch ligand is a binding factor for Notch receptor. For example, Notch-1, Notch-2, Notch-3 and Notch-4 have been identified as Notch receptors in human. Notch ligand can be appropriately selected according to the kind of the cell to be produced. It is also preferable to use a Dlk family member as the Notch ligand. Examples of the Dlk family member include Delta-1, Delta-3, Delta-4, Delta-like 1(Dlk1), Dlk3 and Dlk4. Notch ligand may be modified. Notch ligand may also be used as a naturally occurring component or recombinant protein. For the details of the Notch ligand, refer to, for example, Furie et al, Cell 53: 505-518 (1988); Suzuki et al, EMBO J. 6: 1891-1897 (1987); US-A-20040171148.

For the culture in the method of the present invention, a certain factor can be appropriately used according to the kind of the stem cell to be used and the differentiated cell to be produced. For example, when NKT cell is to be produced, a factor such as Flt3 ligand, IL-7 and IL-15 and the like are used. The concentration of these factors is not particularly limited as long as differentiation into NKT cell can be induced and, for example, they can be used at a concentration of about 5 - 10 ng/ml.

Other culture conditions can be determined as appropriate. For example, the culture temperature is not particularly limited and is about 30 - 40°C, preferably about 37°C. The CO₂ concentration is, for example, about 1 - 10%, preferably about 5%.

The cell produced by the method of the present invention may be any cell that can be yielded by differentiation of a stem cell and, for example, immunocytes including B cell lineage cells such as B cell and the like, e.g., CD19⁺ cell, granulocyte lineage cells such as granulocyte and the like, e.g., Gr-1⁺ cell, T cell lineage cells such as T cell and the like, e.g., CD4⁺ and/or CD8⁺ cell, NKT cell lineage cells such as NKT cell and the like, e.g., TCRVβ⁺/αGalCer-presenting CD1d⁺ cell, and the like can be mentioned. More specifically, when the cell produced by the method of the present invention is NKT cell, the produced NKT cell can be a functional NKT cell which can, for example, interact with αGalCer-presenting dendritic cell to produce cytokines such as IFN-γ, IL-4 and the like, exhibit an immunoadjuvant effect (e.g., promotion of maturation of dendritic cell, induction of antigen specific T cell), and further, exhibit a cancer cell growth suppressive action. The method of the present invention is superior in that it can produce such functional cells highly efficiently.

The contents disclosed in any publication cited in the present specification, including patents and patent applications, are hereby incorporated in their entireties by reference, to the extent that they have been disclosed herein.

The present invention is explained in more detail in the following by referring to Examples, which are not to be construed as limitative.

### Examples

(materials and methods)

### ntES cells

As the nuclear transfer (nt) ES cell derived from NKT cell, which is one type of peripheral T lymphocyte, the cell of clone 7 was used from among the ES cells established earlier (see WO2006/018998) from NKT cells by direct nuclear transplantation method.

### OP9 cell medium

The OP9 cell medium used for cell culture was prepared by sterilizing a medium prepared to contain 10 g of αMEM (Gibco), 2.2 g of sodium bicarbonate (Wako Pure Chemical Industries, Ltd.), penicillin, streptomycin (Gibco BRL) and 20%(v/v) fetal bovine serum (FCS) (Equitech Bio Inc, Kerrville, TX) per 1 litter of MilliQ water, by passing the medium through a bottle top filter (0.45 µm) (Corning).

### OP9 cells and OP9-dlk cells

OP9 cells used were cells (RCB1124) purchased from Riken BioResourse Center Riken Cell Bank. OP9 cells (OP9-dlk cells) forced to express delta-like 1, a notch ligand, were prepared according to a publication (Schmitt et al., Nature Immunology 5: 410-417 (2004)). Mouse delta-like 1 was acquired by the RT-PCR method from the cDNA library of the murine thymus. dlk was inserted into the upstream of retrovirus IRES having an IRES-human NGFR structure, transduced into OP9 cells, and the expression was confirmed using an anti-human NGFR monoclonal antibody.

### Maintenance of OP9 cells, OP9-dlk cells and ntES cells

ntES cells (clone 7) derived from NKT cell were maintained by culturing on mouse embryonic fibroblasts in DMEM medium (Sigma) containing 20% FCS, penicillin, streptomycin (Gibco BRL) and LIF (Chemicon, 2x10³ U/ml). OP9 cells and OP9-dlk cells were maintained by dividing the cells into 1/4 at 90% confluent and passaging the divided cells. When subjected to coculture with ntES cell, these cells were maintained for two days or longer after becoming full confluence and then used (hereinafter to be referred to as OP9 cell and OP9-dlk cell for differentiation induction).

### Confirmation of NKT cell appearance

NKT cell was confirmed by FACS (Fluorescence activated cell sorting) method. To be precise, a cell stained with both FITC-labeled anti-mouse TCRVβ (PharMingen) and PE-labeled α-galactosylceramide (αGalCer) pulsed CD1d dimmer molecule (PharMingen) (αGalCer-CD1d dimer⁺) was defined as NKT cell. As a negative control, a vehicle molecule-pulsed PE-labeled CD1d dimmer molecule was used.

### Example 1: differentiation induction of ntES cells

Clone 7 was cultured in a 3.5 cm culture dish in the presence of LIF to 20-30% confluent, and washed twice with phosphate buffer (PBS). TE (trypsin/EDTA, Sigma) was added and the mixture was stood still in an incubator at 37°C for 5 min. The mixture was stirred well with an electric pipet and centrifuged at 500xg for 5 min. The supernatant was removed and the cells were counted. The cells were suspended in OP9 cell culture medium, and spread on OP9 cells or OP9-dlk cells for differentiation induction (each 1.0x10⁵ cells/10 cm culture dish). Thereafter, the cells were stood still in an incubator at 37°C, 5% CO₂ for 3 days and the culture medium was exchanged. Culture was continued for two more days to induce mesoderm cells. The mesoderm cells were separated from OP9 cells or OP9-dlk cells by TE treatment. Cocultured cells were washed twice with 4 ml of PBS, 4 ml of TE was added per one culture dish. The mixture was stood still in an incubator at 37°C, 5% CO₂ for 5 min, neutralized with 8 ml of OP9 cell culture medium, stirred well with an electric pipet and stood still again in an incubator at 37°C, 5% CO₂ for 30 min. The supernatant was recovered, the cells were counted, 1.0x10⁶ mesoderm cells were newly spread on OP9 cells or OP9-dlk cells (10 cm culture dish) for differentiation induction. From this time point, mouse flt3-ligand (flt3L) (R&D) was added at a concentration of 5 ng/ml to OP9 cell culture medium. Thereafter, the culture medium and flt3L were exchanged every 2 days. From day 7 after the start of coculture, mouse IL-7 (R&D), human IL-15 (R&D, only during coculture with OP9-dlk cells) were also added simultaneously to the culture medium each at a concentration of 5 ng/ml, and exchanged together with the culture medium every two days. In this state, blood and lymphocyte lineage stem cells (hematoblasts, lymphoblasts) were allowed to emerge, after which medium and cytokine exchanges were continued until the cell count reached several million. The cells were newly spread in a 6-well plate containing OP9 cells or OP9-dlk cells for differentiation induction at a concentration of 1x10⁶ per well, and cobble stones (immature T, B cell colony) were allowed to form (emerged in 13-16 days after start of coculture). Medium and cytokine exchanges were continued as mentioned above until the cobble stones completely covered the OP9 cells or OP9-dlk cells. The cobble stone cells were stirred well with an electric pipet, separated from OP9 cells or OP9-dlk cells, and newly spread on OP9 cells or OP9-dlk cells for differentiation induction (10 cm culture dish). Culture was continued while exchanging the medium and cytokine every two days. When the cell count exceeded 1x10⁸ per 10 cm culture dish, the cells were passaged on different OP9 cells or OP9-dlk cells for differentiation induction (10 cm culture dish) to allow growth.
In this experiment, whether the experiment progressed normally was confirmed using, in parallel to the ntES cells (clone 7) obtained by the nuclear transplantation technique, ES cells established from conventional fertilized egg. Namely, by the use of the latter cells, CD4⁺CD8⁺ double positive T cells (DP cells) should be produced on the OP9-dlk cells and B cells should be produced on the OP9 cells.

### 1) Induction of differentiation into B cell and granulocyte using OP9 cells

These cells were confirmed using anti-mouse CD19 and anti-mouse Gr-1 monoclonal antibody (PharMingen). On OP9 cells, CD19⁺ (B cell marker) cells and Gr-1⁺ (granulocyte marker) cells emerged from both clone 7 and conventional ES cells, whereas these cells were not produced when OP9-dlk cells was used.

### 2) Induction of differentiation into NKT and T cell using OP9-dlk cells

As shown in the previous report (Schmitt et al., Nature Immunology 5: 410-417 (2004)), when conventional ES cells (M1) were cultured or differentiated on OP9-dlk cells, CD4⁺CD8⁺ DP cells were produced. On the other hand, when clone 7 was cultured or differentiated on OP9-dlk cells, cells such as CD8⁺ SP cells, CD4⁻CD8⁻ cells and the like emerged in addition to these DP cells. At that time, when the cells were stained with anti-mouse TCRVβ and αGalCer-CD1d dimer, not less than 80% of the lymphocytes derived from clone 7 were NKT cells (TCRVβ⁺/αGalCer-CD1d dimer⁺). On the other hand, when a vehicle molecule-pulsed CD1d dimer molecule was used as a negative control, these cells were not stained. In addition, such cells did not emerge in the DP cells prepared from conventional ES cells. From the above results, it was clarified that NKT cells defined by TCRVβ⁺ αGalCer-CD1d dimer⁺ could be produced with very high efficiency when ES cells (ntES cells) prepared by nuclear transplantation of the nucleus of peripheral NKT cells after gene rearrangement were used.

### 3) Expression of co-receptor on NKT cell surface

Most of the NKT cells defined by TCRVβ⁺/αGalCer-CD1d dimer⁺ appeared in the murine thymus are NK1.1⁺, CD44^{high}, CD69⁺ and CD4⁺, and the CD8⁺ population is rare. However, expression of NK1.1 or CD69 was hardly observed on the surface of the NKT cells produced from clone 7 and defined by TCRVβ⁺/αGalCer-CD1d dimer⁺, and only a few cells expressed CD44. Moreover, cells expressing CD4 hardly existed in the cells, about half of the number thereof was CD8 single positive, and the rest was CD4⁻ CD8⁻. The repertoire of the TCRVβ chain used was Vβ8.

### 4) Change in gene program along with differentiation induction of clone 7

To track changes at a gene level that occur along with the differentiation induction of ES cells and ntES cells (clone 7) differentiated on OP9 cells and OP9-dlk cells, the expression of mRNA of a factor important for the differentiation into T, B cells was studied by the semiquantitative RT-PCR method. mRNA (vα14-jα281) specific to NKT cell defined by TCRVβ⁺/αGalCer-CD1d dimer⁺ was not observed on OP9 cells, but emergence of the mRNA was confirmed on day 15 of culture on OP9-dlk cells (Fig. 1). In addition, expression of rag, necessary for forming repertoires of B cell receptor and T cell receptor (TCR), emerged on OP9 cells on day 19 of culture and was confirmed on OP9-dlk cells from day 12 of culture. Furthermore, expression of gata3, essential for programming of T cell differentiation, was observed under the same conditions. In contrast, expression of Igα and λ5, considered to be necessary for differentiation or maturation into B cell, was observed only on OP9 cells both in conventional ES cells and ntES cells (clone 7). From the above results, it was confirmed that dlk is essential for the differentiation of embryonic stem cell into T cell shown in the previous report (Schmitt et al., Nature Immunology vol. 5, p410-417 (2004)), and it was clarified that NKT cell defined by TCRVβ⁺/αGalCer-CD1d dimer⁺ is developed or differentiated under almost the same conditions as for the differentiation into T cell.

### Example 2: Functional analysis of NKT cell induced from ntES cell

### 1) in vitro cytokine production function

Whether or not these NKT cells defined by TCRVβ⁺/αGalCer-CD1d dimer⁺ indeed function was verified. These cells prepared from mouse interact with αGalCer-presenting dendritic cell (DC) and produce cytokines such as IFN-γ, IL-4 and the like. Thus, TCRVβ⁺/αGalCer-CD1d dimer⁺ cells induced from clone 7 were cocultured with αGalCer-presenting DCs, and the concentration of each cytokine released in a culture medium was quantified by the ELISA method. OptEIA mIL-10 ELISA kit (Japan Becton, Dickinson) was used for mouse IL-10, and R&D Systems, Duosets (DY485, DY404, DY413) were used for mouse IFN-γ, IL-4, IL-13, respectively. DCs were purified from splenocytes of NKT cell deficient mouse (C57BL/6 background TCR Jα281 chain deficient mouse) using mouse CD11c-beads [CD11c(N418) microbeads, Miltenyi]. The purity after passing a magnetic column using the beads was not less than 96%. As a control, DP cells differentiated from conventional ES cells were cocultured with αGalCer-presenting DC and these CD11c⁺ cells were also used for quantification of cytokine in the supernatant. As a result of the experiment, IFN-γ and IL-4 were not produced by culturing these DP cells, but the production of both cytokines was observed when TCRVβ⁺/αGalCer-CD1d dimer⁺ cells were used (Figs. 2 and 3). Moreover, since production of these cytokines was not observed in the culture of αGalCer-presenting DCs alone, it was concluded that IFN-γ and IL-4 were released from TCRVβ⁺/αGalCer-CD1d dimer⁺ cells.

### 2) in vivo immunoadjuvant effect of NKT cell derived from ntES cell

As shown above, TCRVβ⁺/αGalCer-CD1d dimer⁺ cells induced from clone 7 showed an ability to produce cytokines such as IFN-γ, IL-4 and the like in vitro in response to appropriate stimulation. In addition, NKT cell is known to mature dendritic cell during activation by the agonist αGalCer, exhibit an immunoadjuvant effect in vivo, and induce antigen specific T cells (Fujii et al., 2003, J. Exp. Med. 198, p267-279). The threshold of the number of NKT cells necessary to enable induction of acquired immunity as studied based on whether immunoadjuvant ability is exhibited by transfusion of C57BL/6-derived (auto) or BALB/c-derived (allo) NKT cells into an NKT cell deficient mouse (C57BL/6 background TCR Jα281 chain deficient mouse), followed by simultaneous administration of OVA protein antigen and αGalCer (2 µg/mouse). Thereafter, whether an adjuvant effect can be induced by ntES cell-derived NKT cells we established was studied and compared with the results by auto- or allo-NKT cell transfusion.
To be specific, to allow effective uptake of antigen by DC in the body, cell-associated ovalbumin (OVA) (2x10⁷ cells/mouse) introduced into the splenocyte of TAP gene deficient mouse at a high osmotic pressure (10 mg/ml) was simultaneously injected intravenously, and 7 days later, the mouse was euthanized by cervical dislocation and splenic mononuclear cells were prepared. The cells were stimulated again in the presence of OVA peptide for 6 hr, and production of IFN-γ in CD8⁺ cells was examined using anti-mouse CD8α antibody (PharMingen) and anti-IFN-γ antibody. As a result, antigen specific production of IFN-γ was confirmed in CD8⁺ cell by restimulation with OVA in mouse co-administered with αGalCer and cell-associated OVA, but in mouse immunized with αGalCer or OVA alone, IFN-γ was not detected in the same cell even by OVA restimulation. The OVA specific IFN-γ production is NKT cell-dependent (Fujii et al., 2003, J. Exp. Med. 198, p267-279), and is considered to be recoverable by previous administration of NKT cells before immunization of NKT cell deficient mouse with the antigen and αGalCer as mentioned above. Thus, NKT cells were purified from C57BL/6 and BALB/c mouse and adoptively transferred to NKT cell deficient mouse. When syngenic (derived from C57BL/6) NKT cells in the number of 2×10⁶ were transferred, CD8⁺ cell in the splenocyte immunized with αGalCer and OVA produced IFN-γ by OVA restimulation, but the incidence was 0.38%. In contrast, when the same number of allogenic NKT cells (derived from BALB/c) was transferred, IFN-γ was detected in 0.13% of splenic CD8⁺ cells. This indicates the possibility of rejection of allogenic NKT cells by the host after administration as compared to syngenic cells. When the mouse was transfused with TCRVβ⁺/αGalCer-CD1d dimer⁺ cells (2×10⁶) derived from ntES cells (clone 7) we induced and thereafter immunized with OVA and αGalCer, 0.38% of IFN-γ producing CD8⁺ T cells was observed OVA dependently, and antigen specific CD8⁺ T cells having almost the same titer as with syngenic cells were observed.

### 3) Inhibitory effect of ntES cell-derived NKT cell on cancer growth

The experiment of the above-mentioned 2) has clarified that ntES cell-derived TCRVβ⁺/αGalCer-CD1d dimer⁺ cell exhibits immunoadjuvant ability. Then, whether this function leads to a cancer metastasis preventive function of the induced antigen specific T cell was investigated. The ntES cell-derived TCRVβ⁺/αGalCer-CD1d dimer⁺ cells (2x10⁶) were transferred to NKT cell deficient mouse (C57BL/6 background TCR Jα281 chain deficient mouse) by intravenous injection, and the mouse was immunized with αGalCer and OVA one hour later. One week later, EG7 cells (2x10⁶), which are cancer cells expressing OVA antigen (EL4 lymphoma cell expressing OVA antigen), or EL4 lymphoma cells (2x10⁶), which are different cancer cells not expressing the antigen, were subcutaneously administered, and the effects on tumor formation and growth were studied by comparison based on the tumor diameter measured with time. By this experiment, whether induction of IFN-γ producing antigen-specific acquired immunity exhibits an antitumor effect can be judged. The results are shown in Figs. 4 and 5.
By the evaluation at 15 days later, in 4 out of 5 mice administered with ntES cell-derived TCRVβ⁺/αGalCer-CD1d dimer⁺ cells, EG7 cells were rejected and the growth of cancer cell was suppressed. Although the tumor diameter increased in one of them, the increase was very small.
As a result of the experiments, the growth of cancer cell was suppressed in an antigen specific manner in the mouse transfused with ntES cell-derived NKT cells, whereas cancer growth could not be suppressed in the mouse without transfusion of the cells or the mouse transfused with cancer cells with different immunization antigen. From the above experimental results, it was demonstrated that the ntES cell-derived NKT cells inhibit growth of cancer cells by exhibiting an antigen-specific immunoadjuvant effect.

This application is based on a patent application No. 2006-259295 filed in Japan (filing date: September 25, 2006), the contents of which are incorporated in full herein by this reference.

## Claims

1. A method of producing a functional differentiated cell having a post-rearrangement genotype of a particular antigen receptor gene, which comprises culturing a stem cell having the genotype in a medium to give a differentiated cell derived from the stem cell.

2. The method of claim 1, wherein the stem cell having the genotype is established by transplanting the nucleus of a cell having the genotype.

3. The method of claim 1 or 2, wherein the stem cell is an ES cell.

4. The method of any one of claims 1 to 3, wherein the antigen receptor is a T cell receptor.

5. The method of claim 4, wherein the T cell receptor is an NKT cell specific T cell receptor.

6. The method of claim 1, wherein an ES cell established by transplanting the nucleus of an NKT cell to an enucleated oocyte is cultured in a medium to give a cell of NKT cell lineage.

7. The method of claim 6, wherein the cell of NKT cell lineage is an NKT cell.

8. The method of claims 1 to 7, wherein the culture is performed in the presence of a feeder cell.

9. The method of claim 8, wherein the feeder cell expresses a Notch ligand.

10. The method of claim 9, wherein the Notch ligand is a delta family member.

11. The method of claim 10, wherein the delta family member is Dlk1.

12. The method of any one of claims 8 to 11, wherein the culture is performed using one or more factors selected from the group consisting of Flt3 ligand, IL-7 and IL-15.

13. A differentiated cell obtainable by the method of any one of claims 1 to 12.

14. The cell of claim 13, wherein the differentiated cell is an NKT cell.
